(19) Europäisches Patentamt
European Patent Office
Office européen des brevets

(11) **EP 4 332 210 A1**

(12) **EUROPEAN PATENT APPLICATION**
published in accordance with Art. 153(4) EPC

(43) Date of publication:
**06.03.2024  Bulletin 2024/10**

(21) Application number: **22876677.0**

(22) Date of filing: **24.08.2022**

(51) International Patent Classification (IPC):
*C12M 1/12* (2006.01)       *C12N 11/06* (2006.01)
*C12N 11/08* (2020.01)      *A61K 9/16* (2006.01)
*A61K 35/12* (2015.01)      *A61K 8/02* (2006.01)
*A61K 8/73* (2006.01)       *A61K 8/65* (2006.01)
*A61K 8/98* (2006.01)

(52) Cooperative Patent Classification (CPC):
A61K 8/02; A61K 8/65; A61K 8/73; A61K 8/98;
A61K 9/16; A61K 35/12; A61L 27/20; A61Q 19/00;
C12M 1/12; C12N 11/06; C12N 11/08

(86) International application number:
**PCT/KR2022/012636**

(87) International publication number:
**WO 2023/054902 (06.04.2023 Gazette 2023/14)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority:  **30.09.2021  KR 20210130366**

(71) Applicant: **LG Chem, Ltd.**
**Yeongdeungpo-gu
Seoul 07336 (KR)**

(72) Inventors:
• **KIM, Yunseop
Daejeon 34122 (KR)**
• **KIM, Jee Seon
Daejeon 34122 (KR)**

(74) Representative: **Plasseraud IP
66, rue de la Chaussée d'Antin
75440 Paris Cedex 09 (FR)**

(54) **MICROCARRIER, CELL COMPLEX, AND MEDICAL COMPOSITION, COSMETIC COMPOSITION, MEDICAL PRODUCT, AND COSMETIC PRODUCT CONTAINING SAME**

(57)    The present invention relates to a micro carrier comprising: polymer micro particles having a core-shell structure which comprises: a core containing a first biocompatible polymer, a metal ion, and an organic crosslinking agent containing at least one reactive functional group; and a shell surrounding the whole or a part of the core and containing a second biocompatible polymer, a metal ion, and an organic crosslinking agent containing at least one reactive functional group, and a cell adhesion-inducing layer formed on the surface of the polymer micro particles, a cell composite, medical composition, cosmetic composition, medical articles and cosmetic articles using the same.

【FIG. 1】

**Description**

**[TECHNICAL FIELD]**

CROSS-REFERENCE TO RELATED APPLICATION(S)

This application claims the benefit of Korean Patent Application No. 10-2021-0130366 filed on September 30, 2021 with the Korean Intellectual Property Office, the entire contents of which are incorporated herein by reference.

The present invention relates to a micro carrier that not only can achieve excellent mechanical strength and stability, but also can be injected into the body immediately after 3D culture without a cell detachment process and can provide a stable environment for adherent cells, thereby increasing cell viability and contributing to a high implantation rate in the body, and a cell composite, medical composition, cosmetic composition, medical articles and cosmetic articles using the same.

**[BACKGROUND ART]**

As the biopharmaceutical and regenerative medical fields expand, there is a growing demand for large-scale cell culture technology capable of efficiently producing cells, tissues, microorganisms, and the like.

Adherent cells are cultured using micro carriers in a 3D bioreactor. The cells, culture medium and microcarrier are placed in the bioreactor, and the culture medium is agitated to bring the cells into contact with the micro carriers, so that the cells are adhered onto the surface of the micro carrier and cultured. Since the micro carrier used at this time provides a high surface area/volume ratio to which cells can attach and grow, it is suitable for large-scale culture of cells. However, when the adherent cells are expanded and cultured using a micro carrier, a process of recovering cells through a cell detachment process is essentially accompanied after the culture is terminated. The cell detachment process induces cell detachment by using a proteolytic enzyme or changing the temperature, but there is a problem that adding such a detachment process can increase manufacturing costs, reduce economics, and cause cell damage.

The development of new materials and processes for solving these problems is steadily progressing, especially, in the case of cell therapeutic agents that inject cells in vivo, attempts have been conducted to ensure the biocompatibility of micro carriers for culturing cells and to eliminate the separation and purification process. In this case, particles are required that achieve strength that can withstand the stress applied by the fluid surrounding the carrier during the culturing process and after injection into the body.

In addition, in the case of a transdermal drug delivery technology in which a micro carrier that has collected drugs or physiologically active materials is mounted on a micro needle and delivered, the micro carrier should use a polymer suitable for application to a living body, and must have sufficient strength so that particles do not deform in the process of passing through the stratum corneum layer of the skin. The micro carriers that have stably penetrated the skin allow local or systemic delivery of loaded drugs and become possible to act on the required lesion.

Hyaluronic acid, which is mainly used as a biocompatible material, is composed of N-acetyl-D-glucosamine and D-glucuronic acid, and is a biopolymer material in which the repeating units are linearly connected. This is abundantly present in the vitreous humor of the eye, synovial fluid of joints, cockscomb, and the like. Hyaluronic acid is often used as a bioinjectable material due to its excellent biocompatibility and viscoelasticity, but hyaluronic acid itself is easily decomposed in vivo or under conditions such as acids and alkalis, which limits its use. Further, when applied to micro carriers, hyaluronic acid exhibits a negative charge in the biological pH range, which causes a problem in that cell adhesion is significantly reduced.

Further, gelatin is a polymer obtained by hydrolyzing collagen, which is a biological connective tissue, and is often used as a scaffold for cell culture. Cells can be collected or cultured, but it is weak in strength and sensitive to temperature, and thus efforts have been made to improve its strength by introducing functional group s by chemical methods.

There is a need to develop micro carriers or polymer micro particles having excellent physical properties and excellent stability such as physical strength and stability against heat and enzymes, while being biocompatible.

Further, in the process of large-scale expansion of adherent cells using micro carriers, a spherical micro carrier that can increase surface efficiency has been developed to overcome the limitations of existing two-dimensional culture, and utilized for three-dimensional expansion culture. In the prior art, in which cells are recovered through a cell detachment process after the completion of culture, there are problems of an increase in manufacturing cost and cell damage due to the addition of a detachment process.

Further, in the case of injectable cell therapeutic agent, the cells are mainly injected into the affected area in a state of floating on the liquid phase, whereby in the case of adherent cells, there was a limitation in that the transplantation rate and cell viability are lowered due to an unstable environment for survival and an immune response in the body.

Therefore, there is a need to develop a micro carrier that can be injected into the body immediately after 3D culture without a cell detachment process and can provide a stable environment for adherent cells, thereby increasing cell viability and contributing to a high implantation rate in the body.

## [DETAILED DESCRIPTION OF THE INVENTION]

### [Technical Problem]

It is an object of the present invention to provide a micro carrier that not only can achieve excellent mechanical strength and stability, but also can be injected into the body immediately after 3D culture without a cell detachment process and can provide a stable environment for adherent cells, thereby increasing cell viability and contributing to a high implantation rate in the body.

It is another object of the present invention to provide a cell composite comprising the micro carrier.

It is another object of the present invention to provide a medical composition comprising the micro carrier or the cell composite.

It is a further object of the present invention to provide a cosmetic composition comprising the micro carrier or the cell composite.

It is still another object of the present invention to provide medical articles comprising the medical composition.

It is still further another object of the present invention to provide cosmetic articles comprising the cosmetic composition.

### [Technical Solution]

In one aspect, there is provided a micro carrier comprising: polymer micro particles having a core-shell structure which comprises: a core containing a first biocompatible polymer, a metal ion, and an organic crosslinking agent containing at least one reactive functional group; and a shell surrounding the whole or a part of the core and containing a second biocompatible polymer, a metal ion, and an organic crosslinking agent containing at least one reactive functional group, and a cell adhesion-inducing layer formed on the surface of the polymer micro particles.

In another aspect, there is provided a cell composite comprising: the micro carrier; and cells adhered onto the surface of the micro carrier.

In another aspect, there is provided a medical composition comprising the micro carrier or the cell composite.

In a further aspect, there is provided a cosmetic composition comprising the micro carrier or the cell composite.

In still another aspect, there is provided a medical article comprising the medical composition.

In still further another aspect, there is provided a cosmetic article comprising the cosmetic composition.

A micro carrier, a cell composite, medical composition, cosmetic composition, medical articles and cosmetic articles using the same according to specific embodiments of the present invention will be described in more detail below.

Unless explicitly stated herein, the technical terms used herein are for the purpose of describing specific embodiments only and is not intended to limit the scope of the invention.

The singular forms "a," "an" and "the" used herein are intended to include plural forms, unless the context clearly indicates otherwise.

It should be understood that the terms "comprise," "include", "have", etc. are used herein to specify the presence of stated feature, region, integer, step, action, element and/or component, but do not preclude the presence or addition of one or more other feature, region, integer, step, action, element, component and/or group.

Further, the terms including ordinal numbers such as "a first", "a second", etc. are used only for the purpose of distinguishing one component from another component, and are not limited by the ordinal numbers. For instance, a first component may be referred to as a second component, or similarly, the second component may be referred to as the first component, without departing from the scope of the present invention.

As used herein, the (co)polymer includes both a polymer or a copolymer, the polymer means a homopolymer consisting of a single repeating unit, and the copolymer means a composite polymer containing two or more repeating units.

While the present invention can be modified in various ways and take on various alternative forms, specific embodiments thereof are illustrated and described in detail below. However, it should be understood that there is no intent to limit the present invention to the particular forms disclosed, but on the contrary, the intention is to cover all modifications, equivalents, and alternatives falling within the spirit and scope of the invention as described.

As used herein, the term "micro particles" means that the cross section of the particle is circular or elliptical, and the minor axis/major axis ratio (spheroidization ratio) of the particle is in the range of 0.7 to 1.0. The length of the minor axis and the major axis of the particle can be derived by taking an optical photograph of the particle and calculating the average value of any 30 to 100 particles in the optical photograph.

As used herein, a particle diameter Dn means a particle diameter at the n volume% point in the cumulative distribution of particles according to the particle diameter. That is, D50 is a particle diameter at the 50% point in the cumulative distribution of particles when particle diameters of particles are accumulated in ascending order, D90 is a particle diameter at the 90% point in the cumulative distribution of particles according to the particle diameter, and D10 is a particle diameter at the 10% point in the cumulative distribution of particles according to the particle diameter.

The Dn may be determined using a laser diffraction method. In detail, powders to be measured are dispersed in dispersed medium, and then introduced into a commercially available laser diffraction particle size analyzer (Horiba LA-960), and when the particles pass through a laser beam, a difference in diffraction patterns according to the particle size is measured to calculate a particle size distribution. D10, D50 and D90 can be determined by calculating a particle diameter at the 10%, 50% and 90% point in the cumulative distribution of particles according to the particle diameter in the analyzer. More specifically, the diameter used herein may mean D50.

As used herein, an emulsion means a mixed phase in which one or more of immiscible liquids of an oil phase or an aqueous phase is dispersed in a fine particle state (dispersoid) in another liquid (dispersion medium). Emulsions can be generally divided into macro emulsions, micro emulsions, and nano emulsions depending on the particle size of the dispersed phase.

Now, the present invention will be described in more detail.

**1. Micro carrier**

According to one embodiment of the invention, there can be provided a micro carrier comprising: polymer micro particles having a core-shell structure which comprises: a core containing a first biocompatible polymer, a metal ion, and an organic crosslinking agent containing at least one reactive functional group; and a shell surrounding the whole or a part of the core and containing a second biocompatible polymer, a metal ion, and an organic crosslinking agent containing at least one reactive functional group, and a cell adhesion-inducing layer formed on the surface of the polymer micro particles.

The present inventors have conducted intensive research on polymer microparticles, and confirmed through experiments that as an additional crosslinking reaction proceeds after the crosslinking reaction with metal ions as described above, the process efficiency is maximized, and the mechanical strength and cell adhesion of micro particles are remarkably improved, and completed the present invention.

The inventors also confirmed through experiments that as the micro carrier of one embodiment includes a cell adhesion-inducing layer formed on the surface of the polymer micro particles, it can be injected into the body immediately after 3D culture without cell detachment process, and adherent cells form a composite in a state of being stably attached to the micro carrier, which thus increases the cell viability and realizes a high implantation rate in the body, thereby completing the invention.

In one embodiment of the invention, the core may include a polymer matrix in which a first biocompatible polymer is crosslinked via a metal ion and an organic crosslinking agent containing at least one reactive functional group, and the shell may include a polymer matrix in which a second biocompatible polymer is crosslinked via a metal ion and an organic crosslinking agent containing at least one reactive functional group.

Specifically, the polymer matrix may include a first crosslinking region in which the biocompatible polymer is crosslinked via a metal ion; and a second crosslinking region in which the biocompatible polymer is crosslinked via an organic crosslinking agent containing at least one reactive functional group.

The first crosslinking region may mean a crosslinking region formed by a crosslinking reaction between a biocompatible polymer and a metal ion, and the second crosslinking region may include a crosslinking region formed by a crosslinking reaction with an organic crosslinking agent containing at least one reactive functional group instead of a crosslinking reaction between a biocompatible polymer and a metal ion. That is, the polymer micro particles of the embodiment may be produced through a crosslinking reaction using a metal ion and an organic crosslinking agent containing at least one reactive functional group.

Specifically, the biocompatible polymer means a polymer that can be directly injected into a human body in order to deliver effective materials applied to a human body. Specifically, the biocompatible polymer may be one or more polymers selected from the group consisting of hyaluronic acid (HA), carboxymethyl cellulose (CMC), alginic acid, pectin, carrageenan, chondroitin (sulfate), dextran (sulfate), chitosan, polylysine, collagen, gelatin, carboxymethyl chitin, fibrin, agarose, pullulan, polylactide, polyglycolide (PGA), polylactide-glycolide copolymer (PLGA), polyanhydride, polyorthoester, polyetherester, polycaprolactone, polyesteramide, poly(butyric acid), poly(valeric acid), polyurethane, polyacrylate, ethylene-vinyl acetate polymer, acryl-substituted cellulose acetate, non-degradable polyurethane, polystyrene, polyvinyl chloride, polyvinyl fluoride, poly(vinyl imidazole), chlorosulphonate polyolefins, polyethylene oxide, polyvinylpyrrolidone (PVP), polyethylene glycol (PEG), polymethacrylate, hydroxypropylmethylcellulose (HPMC), ethyl cellulose (EC), hydroxypropyl cellulose (HPC), cyclodextrin, copolymers of monomers that form these polymers and cellulose.

More specifically, the biocompatible polymer may be a mixture of hyaluronic acid (HA) and gelatin.

Polymer micro particles produced using only hyaluronic acid, by themselves, are easily decomposed in vivo or under conditions such as acid and alkali, which not only limit their use, but also significantly lower their cell adhesion, and the polymer micro particles produced using only gelatin is significantly reduced in mechanical properties.

Therefore, by using a mixture of hyaluronic acid (HA) and gelatin as a biocompatible polymer, excellent cell adhesion and mechanical properties can be realized at the same time.

Specifically, the first biocompatible polymer may include hyaluronic acid, and the second biocompatible polymer may include gelatin.

As used herein, the hyaluronic acid may include both hyaluronic acid itself and hyaluronate. Thereby, the aqueous hyaluronic acid solution may be a concept that includes all of an aqueous solution of hyaluronic acid, an aqueous solution of hyaluronate, and a mixed aqueous solution of hyaluronic acid and hyaluronate. The hyaluronate may be inorganic salts such as sodium hyaluronate, potassium hyaluronate, calcium hyaluronate, magnesium hyaluronate, zinc hyaluronate, and cobalt hyaluronate, organic salts such as hyaluronic acid tetrabutylammonium, and mixtures thereof.

In one embodiment of the invention, the molecular weight of hyaluronic acid is not particularly limited, but is preferably 10,000 g/mol or more and 5,000,000 g/mol or less in order to realize various physical properties and biocompatibility.

As used herein, the gelatin may mean a protein obtained by treating collagen derived from animals with acid or alkali, followed by extraction.

In one embodiment of the invention, the molecular weight of gelatin is not particularly limited, but is preferably 10,000 g/mol or more and 5,000,000 g/mol or less in order to realize various physical properties and biocompatibility.

Meanwhile, the polymer micro particles may have a core-shell structure. In the core-shell structure, as the polymer matrix contained in the polymer micro particles includes two types or more of biocompatible polymers, it can be realized by a difference in reactivity between a biocompatible polymer, a metal ion and an organic crosslinking agent containing at least one reactive functional group.

In the core-shell structure, the core may contain a polymer matrix, in which hyaluronic acid is crosslinked via a metal ion and an organic crosslinking agent containing at least one reactive functional group, in an amount of more than 50% by volume, more than 60% by volume, more than 70% by volume, or more than or equal to 75% by volume, with respect to the total volume of the polymer matrix contained in the core. Further, it may be contained in an amount of 100 % by volume or less, less than 100% by volume, 95% by volume or less, or 90% by volume or less. It may also contain in an amount of more than 50 % by volume and 100% by volume or less, more than 50% by volume and less than 100% by volume, 60 % by volume or more and 100% by volume or less, 60 % by volume or more and 95 % by volume or less, 70 % by volume or more and 95 % by volume or less, 70 % by volume or more and 90 % by volume or less, or 75 % by volume or more and 90 % by volume or less. That is, the core may contain an excess of hyaluronic acid with respect to the gelatin.

Containing a polymer matrix, in which hyaluronic acid is crosslinked via a metal ion and an organic crosslinking agent containing at least one reactive functional group, in an amount of more than 50% by volume with respect to the total volume of the polymer matrix contained in the core can confirm visually or through a measuring instrument that the corresponding region is distributed in excess of 50% of the total area.

Specifically, calculation of the volume ratio of a polymer matrix, in which hyaluronic acid is crosslinked via a metal ion and an organic crosslinking agent containing at least one reactive functional group, relative to the total volume of the polymer matrix contained in the core is not particularly limited, and it can be calculated by a normal measurement method. For example, it can be confirmed by taking an IR photograph of the hyaluronic acid characteristic peak (1080 $cm^{-1}$) relative to the gelatin characteristic peak (1650 $cm^{-1}$) of the produced polymer micro particles.

In the core-shell structure, the shell may contain a polymer matrix, in which gelatin is crosslinked via a metal ion and an organic crosslinking agent containing at least one reactive functional group, in an amount of more than 50% by volume, 60% by volume or more, 70% by volume or more, or 75% by volume or more with respect to the total volume of the polymer matrix contained in the shell. It may also be contained in an amount of 100% by volume or less, less than 100% by volume, 95% by volume or less, or 90% by volume or less. Further, it may be contained in an amount of more than 50% by volume and not more than 100% by volume, more than 50% by volume and less than 100% by volume, more than 60% by volume and less than 100% by volume, more than 60% by volume and 95% by volume or less, 70% by volume or more and 95% by volume or less, 70% by volume or more and 90% by volume or less, or 75% by volume or more and 90% by volume or less. That is, the shell may contain an excess of gelatin with respect to hyaluronic acid.

Containing a polymer matrix, in which gelatin is crosslinked via a metal ion and an organic crosslinking agent containing at least one reactive functional group, in an amount of more than 50% by volume with respect to the total volume of the polymer matrix contained in the shell can confirm visually or through a measuring instrument that the corresponding region is distributed in excess of 50% of the total area.

Calculation of the volume ratio of a polymer matrix, in which gelatin is crosslinked via a metal ion and an organic crosslinking agent containing at least one reactive functional group, relative to the total volume of the polymer matrix contained in the shell is not particularly limited, and it can be calculated by a normal measurement method. For example, it can be confirmed by taking an IR photograph of the characteristic peak (1650 $cm^{-1}$) of gelatin of the produced polymer micro particles.

The core-shell structure which comprises a core containing 50% by volume or more of a polymer matrix in which hyaluronic acid is crosslinked via a metal ion and an organic crosslinking agent containing at least one reactive functional group and a shell containing 50% by volume or more of a polymer matrix in which gelatin is crosslinked via a metal ion and an organic crosslinking agent containing at least one reactive functional group, can be realized according to physicochemical causes such as solubility, temperature reactivity, ionic bonding properties, and the like. More specifically, in the production process of the polymer micro particles, gelatin, having low solubility in polar solvents such as ethanol and high temperature reactivity, is reduced in fluidity and is fixed and present onto the particle surface to form a shell, whereby a relatively large amount of hyaluronic acid can be distributed inside the particles to form a core.

In particular, in the method for producing polymer micro particles described later, before the step of further crosslinking the polymer crosslinked particles in a polar solvent phase containing an organic crosslinking agent containing at least one reactive functional group, the method includes a step of reacting a mixture containing a biocompatible polymer and a metal ion to form polymer crosslinked particles, whereby the core-shell structure may become clearer due to the ionic bond between a metal ion and a carboxyl group contained in hyaluronic acid.

Meanwhile, the polymer micro particles may have an average diameter in distilled water of 1 $\mu$m or more, 1 $\mu$m or more and 450 $\mu$m or less, 100 $\mu$m or more and 450 $\mu$m or less, or 200 $\mu$m or more and 400 $\mu$m or less, or 300 $\mu$m or more and 400 $\mu$m or less. When the average diameter of the polymer micro particles satisfies the above-mentioned range, cell adhesion and culture performance are excellent.

The average diameter may mean a particle diameter at the 50 volume% point in the cumulative distribution of particles according to the particle diameter.

In the polymer micro particles, based on a cross section having the longest diameter of the polymer micro particles, the thickness of the shell may be 95% or less, 90% or less, 80% or less, 75% or less, 50% or less, 30% or less, 25% or less, or 20% or less of the longest diameter of the polymer micro particles. Also, based on the cross section having the longest diameter of the polymer micro particles, the thickness of the shell may be 0.01% or more, 1% or more, or 5% or more of the longest diameter of the polymer micro particles.

Further, in the polymer micro particles, based on the cross section having the longest diameter of the polymer micro particles, the thickness of the core may be 5% or more, 10% or more, 20% or more, 25% or more, 50% or more, 70% or more, 75% or more, or 80% or more of the longest diameter of the polymer micro particles. Also, based on the cross section having the longest diameter of the polymer micro particles, the thickness of the core may be 99.99% or less, 99% or less, or 95% or less of the longest diameter of the polymer micro particles.

Further, the polymer micro particles may have a spheroidization degree of 0.9 or more and 1.0 or less, 0.93 or more and 1.0 or less, 0.94 or more and 0.99 or less, or 0.94 or more and 0.98 or less. The spheroidization degree can be obtained by taking an optical photograph of the polymer micro particles and calculating the average value of 30 to 100 arbitrary particles in the optical photograph.

Further, the average compressive strength of the polymer micro particles when deformed to a level of 25% of the average diameter of the particles swollen with distilled water for 24 hours or more may be 0.1 mN or more, 0.1 mN or more and 100 mN or less, 0.3 mN or more and 100 mN or less, 0.35 mN or more and 100 mN or less, 0.35 mN or more and 30 mN or less, 0.35 mN or more and 10 mN or less, or 0.35 mN or more and 3 mN or less. The average compressive strength may be a value obtained by dividing the compressive strength by n when the n polymer micro particles are deformed to a level of 25% of the average diameter.

For example, as used herein, the average compressive strength may be a value obtained by dividing the compressive strength by 30 when the 30 polymer micro particles are deformed to a level of 25% of the average diameter.

When the average compressive strength of the polymer micro particles is less than 0.1 mN, there may be a technical problem that the mechanical strength of the polymer micro particles is inferior and the stability is reduced.

Meanwhile, examples of the method for producing the polymer micro particles contained in the micro carrier of the one embodiment is not particularly limited, but for example, a method for producing polymer micro particles, including a step of reacting a mixture containing a biocompatible polymer and a metal ion to form polymer crosslinked particles; and a step of further crosslinking the polymer crosslinked particles in a polar solvent phase containing an organic crosslinking agent containing at least one reactive functional group can be used.

Conventional polymer micro particles are crosslinked after forming a W/O emulsion using oil, and thus, an oil washing process is necessarily accompanied, which causes a technical problem that the process efficiency is not good, and it is difficult to remove residual oil.

Thus, the present inventors have confirmed through experiments that as in the method for producing the polymer micro particles, an additional crosslinking reaction proceeds using an organic crosslinking agent containing at least one reactive functional group after a crosslinking reaction by metal ions, thereby maximizing the process efficiency and remarkably improving the mechanical strength and stability of the micro particles, and completed the present invention.

Specifically, the biocompatible polymer means a polymer that can be directly injected into a human body in order to deliver effective materials applied to a human body. Specifically, the biocompatible polymer may be one or more polymers selected from the group consisting of hyaluronic acid (HA), carboxymethyl cellulose (CMC), alginic acid, pectin, carrageenan, chondroitin (sulfate), dextran (sulfate), chitosan, polylysine, collagen, gelatin, carboxymethyl chitin, fibrin, agarose, pullulan, polylactide, polyglycolide (PGA), polylactide-glycolide copolymer (PLGA), polyanhydride, polyorthoester, polyetherester, polycaprolactone, polyesteramide, poly(butyric acid), poly(valeric acid), polyurethane, polyacrylate, ethylene-vinyl acetate polymer, acryl-substituted cellulose acetate, non-degradable polyurethane, polystyrene, polyvinyl chloride, polyvinyl fluoride, poly(vinyl imidazole), chlorosulphonate polyolefins, polyethylene oxide, polyvinylpyrrolidone (PVP), polyethylene glycol (PEG), polymethacrylate, hydroxypropylmethylcellulose (HPMC), ethyl cellulose (EC), hydroxypropyl cellulose (HPC), cyclodextrin, copolymers of monomers that form these polymers and cellulose.

More specifically, the biocompatible polymer may be a mixture of hyaluronic acid (HA) and gelatin.

Polymer micro particles produced using only hyaluronic acid, by themselves, are easily decomposed in vivo or under conditions such as acid and alkali, which not only limit their use, but also significantly lower their cell adhesion, and the polymer micro particles produced using only gelatin is significantly reduced in mechanical properties.

In this regard, by using a mixture of hyaluronic acid (HA) and gelatin as a biocompatible polymer, excellent cell adhesion and mechanical properties can be realized at the same time.

As used herein, the hyaluronic acid may include both hyaluronic acid itself and hyaluronate. Therefore, the aqueous hyaluronic acid solution may be a concept that includes all of an aqueous solution of hyaluronic acid, an aqueous solution of hyaluronate, and a mixed aqueous solution of hyaluronic acid and hyaluronate. The hyaluronate may be inorganic salts such as sodium hyaluronate, potassium hyaluronate, calcium hyaluronate, magnesium hyaluronate, zinc hyaluronate, and cobalt hyaluronate, organic salts such as hyaluronic acid tetrabutylammonium, and mixtures thereof.

In one embodiment of the invention, the molecular weight of hyaluronic acid is not particularly limited, but is preferably 10,000 g/mol or more and 5,000,000 g/mol or less in order to realize various physical properties and biocompatibility.

As used herein, the gelatin may mean a protein obtained by treating collagen derived from animals with acid or alkali, followed by extraction.

In one embodiment of the invention, the molecular weight of gelatin is not particularly limited, but is preferably 10,000 g/mol or more and 5,000,000 g/mol or less in order to realize various physical properties and biocompatibility.

In the method for producing the polymer micro particles, a mixture of hyaluronic acid (HA) and gelatin may be contained in an amount of 50 parts by weight or more and 500 parts by weight or less, 100 parts by weight or more and 500 parts by weight or less, or 100 parts by weight or more and 300 parts by weight or less with respect to 100 parts by weight of hyaluronic acid (HA).

When gelatin is contained in an amount of less than 50 parts by weight with respect to 100 parts by weight of hyaluronic acid (HA), the cell adhesion of the produced polymer micro particles may be deteriorated, and when gelatin is contained in an amount of more than 500 parts by weight with respect to 100 parts by weight of hyaluronic acid (HA), the mechanical properties of the prepared polymer microparticles may be reduced. That is, the mixture of hyaluronic acid (HA) and gelatin contains 50 parts by weight or more and 500 parts by weight or less of gelatin with respect to 100 parts by weight of hyaluronic acid (HA), polymer micro particles that can realize excellent cell adhesion and mechanical properties at the same time can be produced.

In the method for producing the polymer micro particles, the metal ion may include one selected from the group consisting of iron ion ($Fe^{3+}$), aluminum ion ($Al^{3+}$), copper ion ($Cu^{2+}$), iron ion ($Fe^{2+}$), magnesium ion ($Mg^{2+}$), barium ion ($Ba^{2+}$), calcium ion ($Ca^{2+}$) and the like. More specifically, the metal ion may be an iron ion, an aluminum ion, or a mixture thereof.

In the method for producing the polymer micro particles, the step of reacting the mixture containing the biocompatible polymer and metal ions to form crosslinked polymer particles may include a step of forming an aqueous solution in which the biocompatible polymer is dissolved; a step of adding the compound containing a metal ion to a polar solvent to form a solution containing a metal ion; and a step of by mixing the aqueous solution droplet in which the biocompatible polymer is dissolved and the solution containing a metal ion to form a mixed solution.

Specifically, in the step of forming an aqueous solution in which the biocompatible polymer is dissolved, the aqueous solution in which the biocompatible polymer is dissolved may contain the biocompatible polymer in an amount of 0.01% by weight or more and 10% by weight or less, 0.01% by weight or more and 5% by weight or less, 1% by weight or more and 5% by weight or less, 1% by weight or more and 3% by weight or less, 2% by weight or more and 3% by weight or less, or 2% by weight or more and 2.5% by weight or less.

As in the production method of the polymer micro particles, an additional crosslinking reaction proceeds with an organic crosslinking agent containing at least one reactive functional group after a crosslinking reaction with metal ions, whereby micro particles having excellent mechanical strength and stability can be produced even with a biocompatible polymer content as low as 0.01% by weight or more and 10% by weight or less as described above, as compared to the case of performing a crosslinking reaction after forming a W/O emulsion as in the conventional case.

The step of adding the compound containing a metal ion to a polar solvent to form a solution containing a metal ion is not particularly limited, but for example, the compound containing a metal ion may be dispersed in a polar solvent such as ethanol to form a solution.

Further, in the step of by mixing the aqueous solution droplet in which the biocompatible polymer is dissolved and the solution containing a metal ion to form a mixed solution, the particle size can be appropriately adjusted by an Encapsulator (BUCHI, B-390) device.

By including the step of mixing the aqueous solution droplet in which the biocompatible polymer is dissolved and the solution containing a metal ion to form a mixed solution, a biocompatible polymer can form a crosslinked structure via metal ions while chelating metal ions to the biocompatible polymer.

By including the step of reacting the mixture containing the biocompatible polymer and metal ions to form crosslinked polymer particles as in the method for producing the polymer micro particles, micro particles can be produced in a polar solvent without the use of oil, as compared to the case of performing a crosslinking reaction using only an organic crosslinking agent containing at least one reactive functional group, thereby eliminating the oil washing process and thus realizing the effect of improving process efficiency.

That is, as the biocompatible polymer forms a crosslinked structure via the metal ion while chelating the metal ion to the biocompatible polymer, micro particles can be produced in a polar solvent without the use of oil, as compared to the case of performing the crosslinking reaction using only an organic crosslinking agent containing at least one reactive functional group, thereby eliminating the oil washing process and thus realizing the effect of improving process efficiency.

In addition, the compound containing the metal ion may be contained in an amount of 200 parts by weight or more and 1000 parts by weight or less, 300 parts by weight or more and 1000 parts by weight or less, or 500 parts by weight or more and 1000 parts by weight or less with respect to 100 parts by weight of the biocompatible polymer.

The method for producing the polymer micro particles proceeds an additional crosslinking reaction after the crosslinking reaction by metal ions as described above, whereby even if a compound containing metal ions is added in a small amount of 200 parts by weight or more and 1000 parts by weight or less with respect to 100 parts by weight of the biocompatible polymer, it is possible to prepare polymer micro particles capable of realizing sufficient mechanical strength and spheroidization degree.

When the content of the compound containing the metal ion is added in excess of 1000 parts by weight with respect to 100 parts by weight of the biocompatible polymer, there may be a technical problem that a residual amount of metal ions remains in the crosslinked particles.

The organic crosslinking agent containing at least one reactive functional group may include a crosslinking agent having 1 to 30 carbon atoms containing at least one reactive functional group.

As described above, as an additional crosslinking reaction proceeds using a crosslinking agent having 1 to 30 carbon atoms containing at least one reactive functional group after the crosslinking reaction by metal ions, the process efficiency is maximized and at the same time, the mechanical strength and stability of the micro particles can be significantly improved.

The type of the reactive functional group is not particularly limited, but for example, a hydroxyl group, an epoxy group, a carboxy group, an amino group, a (meth)acrylate group, a nitrile group, a thiol group, an aldehyde group, or a vinyl group may be mentioned.

Specifically, the organic crosslinking agent including at least one reactive functional group may include one or more, or two or more formyl groups or epoxy groups. The formyl group or the epoxy group may be a crosslinkable functional group that reacts with the above-mentioned biocompatible polymer to form crosslinked particles.

In the method for producing the polymer micro particles, examples of the organic crosslinking agent containing at least one reactive functional group are not particularly limited. Specifically, the crosslinking agent may include one selected from the group consisting of glutaraldehyde, 1,4-butandiol diglycidyl ether (BDDE), ethylene glycol diglycidyl ether (EGDGE), 1,6-hexanediol diglycidyl ether, propylene glycol diglycidyl ether, polypropylene glycol diglycidyl ether, polytetramethylene glycol diglycidyl ether, neopentyl glycol diglycidyl ether, polyglycerol polyglycidyl ether, diglycerol polyglycidyl ether, glycerol polyglycidyl ether, tri-methylpropane polyglycidyl ether, 1,2-(bis(2,3-epoxypropoxy)ethylene, pentaerythritol polyglycidyl ether, sorbitol polyglycidyl ether, divinylsulfone, and epichlorohydrin.

More specifically, the organic crosslinking agent containing at least one reactive functional group may be glutaraldehyde or 1,4-butandiol diglycidyl ether (BDDE).

Meanwhile, in the step of further crosslinking the polymer crosslinked particles in a polar solvent phase containing an organic crosslinking agent containing at least one reactive functional group, the organic crosslinking agent containing at least one reactive functional group may be comprised in an amount of 150 parts by weight or more and 1000 parts by weight or less, 200 parts by weight or more and 1000 parts by weight or less, 300 parts by weight or more and 800 parts by weight or less, 400 parts by weight or more and 500 parts by weight or less with respect to 100 parts by weight of the biocompatible polymer.

The method for producing the polymer micro particles proceeds an additional crosslinking reaction after the crosslinking reaction as described above, whereby even if an organic crosslinking agent containing at least one reactive functional group is added in a small amount of 150 parts by weight or more and 1000 parts by weight or less with respect to 100 parts by weight of the biocompatible polymer, it is possible to produce polymer micro particles capable of realizing sufficient mechanical strength and spheroidization degree.

When the content of the organic crosslinking agent containing at least one reactive functional group is added in excess of 1000 parts by weight with respect to 100 parts by weight of the biocompatible polymer, there may be a technical problem that a residual amount of the unreacted crosslinking agent remains in the crosslinked particles.

Meanwhile, in the step of further crosslinking the polymer crosslinked particles in a polar solvent phase containing an organic crosslinking agent containing at least one reactive functional group, the polar solvent is not particularly limited, but for example, it may be one selected from the group consisting of ethanol, N,N-dimethylformamide, N,N-dimethylacetamide, N-methyl-2-pyrrolidone, N-methylcaprolactam, 2-pyrrolidone, N-ethylpyrrolidone, N-vinylpyrrolidone, dimethyl sulfoxide, tetramethylurea, pyridine, dimethyl sulfone, hexamethyl sulfoxide, gamma-butyrolactone, 3-methoxy-N,N-dimethylpropanamide, 3-ethoxy-N,N-dimethylpropanamide, 3-butoxy-N,N-dimethylpropanamide, 1,3-dimethyl-imidazolidinone, ethyl amyl ketone, methyl nonyl ketone, methyl ethyl ketone, methyl isoamyl ketone, methyl isopropyl ketone, cyclohexanone, ethylene carbonate, propylene carbonate, diglyme, 4-hydroxy-4-methyl-2-pentanone, ethylene glycol monomethyl ether, ethylene glycol monomethyl ether acetate, ethylene glycol monoethyl ether, ethylene glycol monoethyl ether acetate, ethylene glycol monopropyl ether, ethylene glycol monopropyl ether acetate, ethylene glycol monoisopropyl ether, ethylene glycol monoisopropyl ether acetate, ethylene glycol monobutyl ether, and ethylene glycol monobutyl ether acetate.

Further, in the step of further crosslinking the polymer crosslinked particles in a polar solvent phase containing an organic crosslinking agent containing at least one reactive functional group, the polar solvent containing an organic crosslinking agent containing at least one reactive functional group may be an alkaline mixed solvent.

That is, the additional crosslinking reaction of the present invention may proceed in an alkaline mixed solvent phase in which an aqueous alkali solution is mixed with a polar solvent. Examples of the aqueous alkali solution are not particularly limited, but may be, for example, an aqueous sodium hydroxide solution.

The polar solvent containing the organic crosslinking agent containing at least one reactive functional group is an alkaline mixed solvent, thereby capable of creating an environment favorable to a nucleophilic substitution reaction ($S_N$ reaction) and improving the efficiency of the crosslinking reaction.

Meanwhile, the micro carrier of one embodiment may include a cell adhesion-inducing layer formed on the surface of the polymer microparticles. The cell adhesion-inducing layer is composed of cell adhesion materials, and allows adherent cells to be stably adhered, spread and cultured.

The cell adhesion-inducing layer may include one or more cell adhesion materials selected from the group consisting of gelatin, collagen, fibronectin chitosan, polydopamine, poly L-lysine, vitronectin, peptide containing RGD, acrylic polymer containing RGD, lignin, cationic dextran and derivatives thereof. That is, the cell adhesion-inducing layer may include a cell adhesion material which includes one selected from the group consisting of gelatin, collagen, fibronectin chitosan, polydopamine, poly L-lysine, vitronectin, peptide containing RGD, acrylic polymer containing RGD, lignin, cationic dextran and derivatives thereof, or a mixture of two or more thereof.

The cell adhesion-inducing layer may be formed on the surface of the polymer micro particles. That is, the cell adhesion-inducing layer may be in direct contact with the surface of the polymer micro particles, or the cell adhesion-inducing layer may be in contact with the surface of another layer that is in contact with the surface of the polymer micro particles.

In one example, the micro carrier includes a cell adhesion-inducing layer formed so as to directly contact the surface of the polymer micro particles, whereby by introducing a cell adhesion-inducing layer on the surface of the micro carrier, the degree of floating of the micro carrier in the culture medium can be adjusted, and the effect of stably adhering and culturing cells can be obtained.

The cell adhesion-inducing layer may have a layer thickness of 1 nm to 10,000 nm, or 10 nm to 1000 nm, or 50 nm to 500 nm, or 80 nm to 200 nm, wherein the layer thickness of the cell adhesion-inducing layer may be obtained by subtracting the radius of the polymer micro particles inside from the overall radius of the micro carrier. The radius means 1/2 value of the diameter. An example of a method for measuring the diameter is not particularly limited, but as an example, it can be measured through confocal fluorescence microscopy, electron transmission microscopy (TEM) or cross-sectional IR, cross-sectional SEM images.

The micro carrier may have an average diameter of 1 $\mu$m to 1,000 $\mu$m, or 10 $\mu$m to 1000 $\mu$m, or 100 $\mu$m to 1,000 $\mu$m, or 100 $\mu$m to 800 $\mu$m. Examples of the method for measuring the diameter are not particularly limited, but as an example, it may be measured through an optical microscope.

Meanwhile, the ratio of the radius of the polymer micro particle to the thickness of the cell adhesion-inducing layer may be 1:0.00001 to 1:0.1, or 1:0.0001 to 1:0.01.

When the ratio of the radius of the polymer micro particles to the thickness of the cell adhesion-inducing layer is less than 1:0.00001, the cell adhesion-inducing layer is too thin compared to the polymer micro particles and thus, it is likely to decrease the degree of adhesion between the cells and the micro carriers during cell culture. When the ratio exceeds 1:0.1, the cell adhesion-inducing layer becomes thicker as compared to the polymer micro particles, which may cause a problem of changing physical properties such as hydrophilicity of the polymer micro particles and thus lowering dispersibility.

The micro carrier may have a cell adhesion of 2000% or more, or 2500% or more, or 2900% or more, or 4000% or less, or 2000% to 4000%, or 2500% to 4000%, or 2900% to 4000% as calculated according to the following Equation.

[Equation]

Cell adhesion = (Number of cells after charging the micro carrier into the cell culture medium and culturing at 37°C for 7 days / Number of cells initially contained in the cell culture medium) X 100.

The cell culture medium, cells, and culture conditions are not particularly limited, and conventionally known various cell culture media, cells, and culture conditions can be applied without limitation. Also, the method for measuring the number of cells is not particularly limited, and conventionally known various methods for measuring the number of cells can be applied without limitation.

As the micro carrier satisfies a cell adhesion of 2000% or more, 2500% or more, 2900% or more, 4000% or less, 2000% to 4000%, or 2500% to 4000%, or 2900% to 4000% as calculated by the Equation, the cell adhesion of the micro carrier is improved, which increases the surface efficiency through three-dimensional expansion culture, and has the advantage of enabling large-scale expansion of adherent cells.

Meanwhile, when the cell adhesion of the micro carrier calculated according to the following Equation does not fall within the above-mentioned range, there is a limit in that the cell adhesion on the surface of the micro carrier is weak, which makes it difficult to sufficiently proceed the three-dimensional expansion culture.

Meanwhile, the micro carrier may further include a primer polymer layer formed on the surface of the polymer micro particles.

That is, a mixed layer of one type of primer polymer layer and one type of cell adhesion-inducing layer may be further included on the surface of the polymer micro particles. In a mixed layer of one type of primer polymer layer and one type of cell adhesion-inducing layer, the laminating order of these is not particularly limited, and a structure in which a cell adhesion-inducing layer is laminated on a primer polymer layer or a structure in which a primer polymer layer is laminated on a cell adhesion-inducing layer is applicable.

Meanwhile, the primer polymer layer functions as an adhesive layer capable of introducing a functional polymer onto the surface of the polymer micro particles, whereby a polymer layer for cell adhesion on the surface of the micro carrier can be effectively introduced and stably maintained during culture.

Examples of the primer polymer layer are not particularly limited, but are catechol derivatives capable of inducing water phase adhesion, and may include any one or more selected from the group consisting of L-dihydroxyphenylalanine (L-DOPA), dopamine, polydopamine, norepinephrine, epinephrine, epigallocatechin and derivatives thereof.

The micro carrier may be a micro carrier for cell culture.

## 2. Cell Composite

According to another embodiment of the present invention, there can be provided a cell composite comprising: the micro carrier; and cells adhered onto the surface of the micro carrier. The contents concerning the micro carrier can include all the contents described above in the one embodiment.

In the cell composite of another embodiment, the composite can be formed in a state where the cells are stably attached to the micro carrier, thereby increasing the cell viability increases and realizing a high implantation rate within a body.

The cell is an adherent animal cell and examples thereof are not particularly limited, but for example, it may be fibroblasts, epithelial cell, osteoblast, chondrocyte, hepatocytes, human-derived cord blood cells, human bone marrow-derived mesenchymal stem cells, CHO (Chinese hamster ovary) cells, kidney cells (HEK293, BHK21, MDCK, vero cell, etc.), or a mixture of two or more thereof.

The density of the cells may be 1.02 g/cm$^3$ or more and less than 1.1 g/cm$^3$.

The cells may be attached onto the surface of the micro carrier. That is, the cell may directly contact the surface of the micro carrier, or the cell may contact the surface of another layer that is in contact with the surface of the micro carrier.

## 3. Medical Composition

According to yet another embodiment of the present invention, there can be provided a medical composition comprising the micro carrier of the one embodiment or the cell composite of the other embodiment. The contents concerning the micro carrier can include all the contents described above in the one embodiment. The contents concerning the cell composite can include all the contents described above in the one embodiment.

The pharmaceutically active material may exist in a state contained in the polymer micro particles.

Examples of the pharmaceutically active material are not particularly limited, and depending on the application use of the polymer micro particles of the one embodiment, an effective material suitable for the use can be applied without limitation. That is, specific examples of the pharmaceutically active material are not limited, and include a drug selected from the group consisting of ampetaminil, arecolin, atrophine, bupranolol, buprenorphine, capsaicin, carisoprodol, chlorpromazine, ciclopirox olamine, cocaine, desipramine, dyclonine, epinephrine, ethosuximide, floxetine, hydromorphine, imipramine, lidocaine, methamphetamine, melproic acid, methylpenidate, morphine, oxibutynin, nadolol, nicotine, nitroglycerin, pindolol, prilocaine, procaine, propanolol, rivastigmine, scopolamine, selegiline, tulobuterol, valproic acid, Donepezil and the like, and a peptide or protein-based drug selected from the group consisting of EPO(Erythropoietin), human growth hormone (hGH), Exenatide, GLP-1(Glucagon-like peptide-1), insulin, CSF(Granulocyte colony-stimulating factor), estrogen, progesterone, parathyroid hormone (PTH), and the like. All pharmacological materials with proven pharmacological effects are applicable without limitation.

The addition amount of the pharmaceutically effective material is also not particularly limited, and the content can be used without limitation depending on the application purpose and object. For example, the effective material may be contained in an amount of 0.0001 parts by weight or more and 1000000 parts by weight or less based on 100 parts by weight of the polymer micro particles, which can be contained without limitation in a small amount or an excess amount relative to the polymer micro particles.

### 4. Cosmetic Composition

According to another embodiment of the present invention, there can be provided a cosmetic composition comprising the micro carrier of the one embodiment or the cell composite of the other embodiment. The contents concerning the micro carrier can include all the contents described above in the one embodiment. The contents concerning the cell composite can include all the contents described above in the one embodiment.

The cosmetically effective material may exist in a state contained in the polymer micro particles.

Examples of the cosmetically effective material are not particularly limited, and depending on the application use of the polymer micro particles of the one embodiment, an effective material suitable for the use can be applied without limitation. That is, specific examples of the cosmetically effective material are not limited, and may include natural extracts, proteins, vitamins, enzymes, antioxidants, and the like, and all materials with proven cosmetic efficacy can be applied without limitation.

The addition amount of the cosmetically effective material is also not particularly limited, and the content can be used without limitation depending on the application purpose and object. For example, the cosmetically effective material may be contained in an amount of 0.0001 parts by weight or more and 1000000 parts by weight or less based on 100 parts by weight of the polymer micro particles, which can be contained without limitation in a small amount or an excess amount relative to the polymer micro particles.

### 5. Medical Article

According to yet another embodiment of the present invention, there can be provided a medical article comprising the medical composition of the other embodiment. The contents concerning the medical composition can include all the contents described above in the one embodiment.

Examples of the medical article are not particularly limited, but in order to realize the characteristics of the present invention, it is suitable when the medical article is used by inserting it into the body or when strength must be maintained for a long period of time, and for example, a body implant, a body insertion type drug delivery system, a transdermal patch, a wound healing agent, and the like can be mentioned.

### 6. Cosmetic Article

According to yet another embodiment of the present invention, there can be provided a cosmetic article comprising the cosmetic composition of the other embodiment of the present invention. The contents concerning the cosmetic composition can include all the contents described above in the one embodiment.

Examples of the cosmetic article are not particularly limited, but in order to realize the characteristics of the present invention, and for example, beauty creams, lotions, hair gels, packs, and the like may be mentioned.

The structure of the cosmetic pack is not particularly limited, but for example, it may include a support, and a cosmetically effective material delivery layer formed on the support and including the polymer micro particles of the other embodiment. Examples of the support include woven fabric, nonwoven fabric, silicone, polyethylene terephthalate, polyethylene, polypropylene, polyurethane, metal mesh, polyester, and the like.

[Advantageous Effects]

According to the present invention, there can be provided a micro carrier that not only can achieve excellent mechanical strength and stability, but also can be injected into the body immediately after 3D culture without a cell detachment process and can provide a stable environment for adherent cells, thereby increasing cell viability and contributing to a high implantation rate in the body, and a cell composite, medical composition, cosmetic composition, medical articles and cosmetic articles using the same.

[BRIEF DESCRIPTION OF THE DRAWINGS]

FIG. 1 is an optical microscope (OM) photograph of the polymer micro particles of Example 1.
FIG. 2 is an IR photograph of the gelatin characteristic peak (1650 cm$^{-1}$) of the polymer micro particles of Example 1.
FIG. 3 is an IR photograph of the hyaluronic acid characteristic peak (1080 cm$^{-1}$) relative to the gelatin characteristic peak (1650 cm$^{-1}$) of the polymer micro particles of Example 1.

[DETAILED DESCRIPTION OF THE EMBODIMENTS]

Hereinafter, the present invention will be described in more detail by way of examples. However, these examples are provided for illustrative purposes only and art not intended to limit the scope of the present invention.

<Example: Production of polymer micro particles and micro carriers for cell culture>

Example 1

(1) Production of polymer micro particles

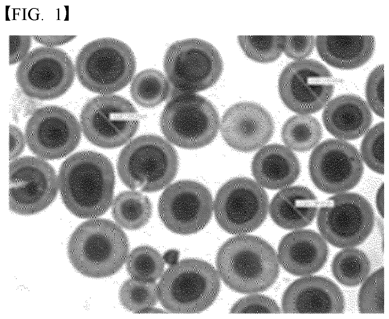

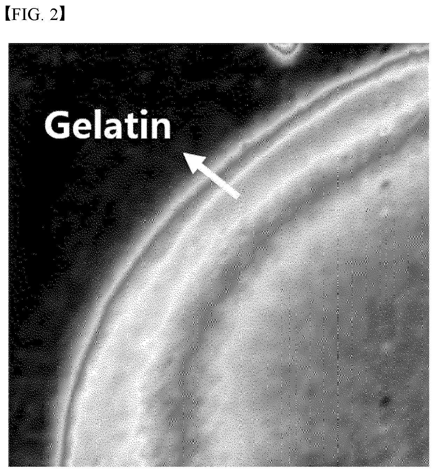

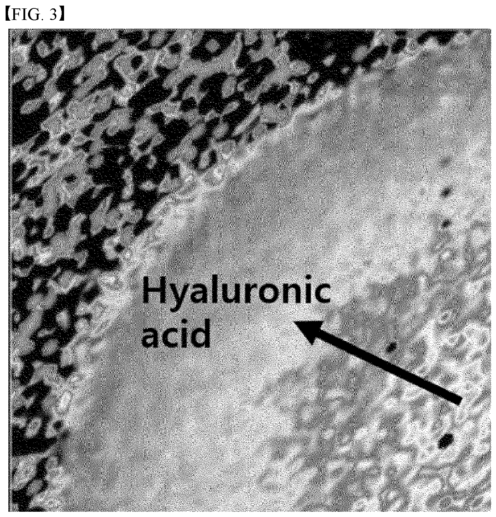

200 mg of hyaluronate (weight average molecular weight: 500 kDa) was dissolved in 0.1N NaOH aqueous solution at a concentration of 2 wt.%, and 250 mg of gelatin (gel strength: 300 g Bloom) was dissolved in distilled water at a concentration of 2.5 wt.%. 10 mL of the respective solutions were mixed to prepare 20 mL, to which the droplet formed by using an Encapsulator device was added to 80 mL of an ethanol solution containing 4 g of FeCl$_3$, which is a compound containing iron ions (Fe$^{3+}$), subjected to a crosslinking reaction at 4°C for 2 hours, and then washed with ethanol to produce crosslinked particles.
2.2 g of 1,4-butanediol diglycidyl ether (BDDE) was mixed with 80% ethanol solution containing 20% of 0.1N NaOH aqueous solution, and then the crosslinked particles were added thereto and subjected to a crosslinking reaction at room temperature for 3 days to produce polymer micro particles. The produced particles were washed with ethanol and distilled water in that order, and then the produced crosslinked particles were recovered using a mesh sieve having a sieve size of 45 $\mu$m. The recovered crosslinked particles were filtered through a mesh sieve having a sieve size of 500 $\mu$m, and the remaining crosslinked particles were analyzed.
An optical microscope (OM) photograph taken of the produced polymer micro particles is shown in FIG. 1.
An IR photograph of the gelatin characteristic peak (1650 cm$^{-1}$) of the produced polymer micro particles is shown in FIG. 2. Through the intensity difference of the characteristic peak of gelatin (1650 cm$^{-1}$), it was confirmed that areas containing gelatin is displayed brightly and thus, gelatin is distributed in the shell of the produced polymer micro particles.
An IR photograph of the hyaluronic acid characteristic peak (1080 cm$^{-1}$) relative to the gelatin characteristic peak (1650 cm$^{-1}$) of the produced polymer micro particles is shown in FIG. 3. It was confirmed that areas containing hyaluronic acid in a high relative amount with respect to gelatin is displayed brightly and thus, hyaluronic acid is distributed in a relatively high amount in the core of the produced polymer micro particles, as compared to gelatin.

(2) Production of micro carriers for cell culture

The recovered particles were immersed in tris buffer (pH 8.0) in which dopamine was dissolved at 1 mg/mL, and coated under stirring at room temperature for 2 hours. After washing excess coating material with ethanol, the particles were filtered on a 45 $\mu$m sieve, and then used as a micro carrier for cell culture.

### Example 2

Polymer micro particles and micro carriers for cell culture were produced in the same manner as in Example 1, except that an ethanol solution containing 4 g of $AlCl_3$, which is a compound containing aluminum ions ($Al^{3+}$), was used instead of an ethanol solution containing 4 g of $FeCl_3$, which is a compound containing iron ions ($Fe^{3+}$).

### Example 3

Polymer micro particles and micro carriers for cell culture were produced in the same manner as in Example 1, except that 2.2 g of 50% glutaraldehyde was added instead of 2.2 g of 1,4-butandiol diglycidyl ether (BDDE).

### <Comparative Example>

### Comparative Example 1: Production of polymer micro particles

Hyaluronate (weight average molecular weight: 500 kDa) and gelatin (gel strength: 300 g Bloom) were dissolved in distilled water at a concentration of 2 wt.% and 20 wt.%, respectively, to prepare 5 ml each, and the solution mixed with these two solutions was mixed with a liquid paraffin solution to prepare a mixed solution containing a micro emulsion. Then, 2.2 g of 1,4-butandiol diglycidyl ether (BDDE) as a crosslinking agent was added to the mixed solution, subjected to a crosslinking reaction at room temperature for 5 days to prepare crosslinked particles. The produced particles washed with acetone, dichloromethane, and distilled water in that order, and then the crosslinked particles produced were recovered using a mesh sieve having a sieve size of 45 $\mu$m. The recovered crosslinked particles were filtered through a mesh sieve with a sieve size of 500 $\mu$m, and the remaining crosslinked particles were analyzed.

### Comparative Example 2: Production of polymer micro particles

Hyaluronate (weight average molecular weight: 500 kDa) was dissolved in 0.1N NaOH aqueous solution at a concentration of 2 wt.%, and gelatin (gel strength: 300 g Bloom) was dissolved in distilled water at a concentration of 2.5 wt.%. 10 mL of the respective solutions were mixed to prepare 20 mL, to which the droplet formed by using an Encapsulator device was added to 80 mL of an ethanol solution containing 4 g of $FeCl_3$, which is a compound containing iron ions ($Fe^{3+}$), subjected to a crosslinking reaction at 4°C for 2 hours, and then washed with ethanol and distilled water to produce crosslinked particles. The produced crosslinked particles were recovered using a mesh sieve having a sieve size of 45 $\mu$m. The recovered crosslinked particles were filtered through a mesh sieve with a sieve size of 500 $\mu$m, and the remaining crosslinked particles were analyzed.

### Comparative Example 3: Production of polymer micro particles

200 mg of Hyaluronate (weight average molecular weight: 500 kDa) was dissolved in 0.1N NaOH aqueous solution at a concentration of 2 wt.%, and 250 mg of gelatin (gel strength: 300 g Bloom) was dissolved in distilled water at a concentration of 2.5 wt.% to prepare 10 ml each. The solution mixed with these two solutions was mixed with a liquid paraffin solution to prepare a mixed solution containing a micro emulsion. Then, 2.2 g of 1,4-butandiol diglycidyl ether (BDDE) as a crosslinking agent was added to the mixed solution, and subjected to a crosslinking reaction at room temperature for 5 days. The reaction product was washed with ethanol, dichloromethane and distilled water in that order, and then the produced crosslinked particles were recovered using a mesh sieve having a sieve size of 45 $\mu$m. The recovered crosslinked particles were filtered through a mesh sieve having a sieve size of 500 $\mu$m, and the remaining crosslinked particles were analyzed.

### Comparative Example 4: Production of polymer micro particles

Hyaluronate (weight average molecular weight: 500 kDa) was dissolved in 0.1N NaOH aqueous solution at a concentration of 2 wt.%, and gelatin (gel strength: 300 g Bloom) was dissolved in distilled water at a concentration of 2.5 wt.% to prepare 5 ml each. The solution mixed with these two solutions was mixed with a liquid paraffin solution to prepare a mixed solution containing a micro emulsion. Then, 2.2 g of 1,4-butandiol diglycidyl ether (BDDE) as a crosslinking agent was added to the mixed solution, and subjected to a crosslinking reaction at room temperature for 5 days. The reaction product was washed with ethanol, dichloromethane and distilled water in that order, and then the produced crosslinked particles were recovered using a mesh sieve having a sieve size of 45 $\mu$m. The recovered crosslinked particles were filtered through a mesh sieve having a sieve size of 500 $\mu$m, and the remaining crosslinked particles were analyzed.

**Comparative Example 5: Production of polymer micro particles**

Hyaluronate (weight average molecular weight: 500 kDa) and gelatin (gel strength: 300 g Bloom) were respectively dissolved in distilled water at a concentration of 2 wt.% and 2.5 wt.% to prepare 5 ml each. The solution mixed with these two solutions was mixed with a liquid paraffin solution to prepare a mixed solution containing a micro emulsion. Then, 2.2 g of 1,4-butandiol diglycidyl ether (BDDE) as a crosslinking agent was added to the mixed solution, and subjected to a crosslinking reaction at room temperature for 5 days. The reaction product was washed with ethanol, dichloromethane and distilled water in that order, and then the produced crosslinked particles were recovered using a mesh sieve having a sieve size of 45 $\mu$m. The recovered crosslinked particles were filtered through a mesh sieve having a sieve size of 500 $\mu$m, and the remaining crosslinked particles were analyzed.

**Comparative Example 6: Production of polymer micro particles**

Alginate and cellulose were respectively dissolved in distilled water at a concentration of 2.5 wt.%. 10 mL of the respective solutions were mixed to prepare 20 mL, to which the droplet formed by using an Encapsulator device was added to 80 mL of an ethanol solution containing 4 g of $CaCl_2$, which is a compound containing calcium ions ($Ca^{2+}$), subjected to a crosslinking reaction at room temperature for 2 hours, and then washed with ethanol to produce crosslinked particles.

2.2 g of 1,4-butandiol diglycidyl ether (BDDE) was mixed with 80% ethanol solution containing 20% of 0.1N NaOH aqueous solution, and then the crosslinked particles were added thereto and subjected to a crosslinking reaction at room temperature for 3 days to produce polymer micro particles. The produced particles were washed with ethanol and distilled water in this order, and then the produced crosslinked particles were recovered using a mesh sieve having a sieve size of 45 $\mu$m. The recovered crosslinked particles were filtered through a mesh sieve having a sieve size of 500 $\mu$m, and the remaining crosslinked particles were analyzed.

**Comparative Example 7: Production of polymer micro particles**

200 mg of hyaluronate (weight average molecular weight: 500 kDa) was dissolved in 0.1 N NaOH aqueous solution at a concentration of 2 wt.%, and 250 mg of gelatin (gel strength: 300g Bloom) was dissolved in distilled water at a concentration of 2.5 wt.%. 10 mL of the respective solutions were mixed to prepare 20 mL, to which the droplet formed by using an Encapsulator (BUCHI, B-390) was added to 80 mL of an ethanol solution containing 4 g of $FeCl_3$, which is a compound containing iron ions ($Fe^{3+}$), subjected to a crosslinking reaction at 4°C for 2 hours, and then washed with ethanol to produce crosslinked particles.

The crosslinked particles were added to 80 mL of ethanol solution containing 4 g of $CaCl_2$, which is a compound containing calcium ions ($Ca^{2+}$), and subjected to a crosslinking reaction at 4°C for 2 hours to produce polymer micro particles. The produced particles were washed with ethanol and distilled water in this order, and then the produced crosslinked particles were recovered using a mesh sieve having a sieve size of 45 $\mu$m. The recovered crosslinked particles were filtered through a mesh sieve having a sieve size of 500 $\mu$m, and the remaining crosslinked particles were analyzed.

**Comparative Example 8: Micro carrier for cell culture**

The polymer micro particles obtained in (1) of Example 1 were used as micro carriers for cell culture.

**<Experimental Example 1>**

For the polymer micro particles produced in Examples and Comparative Examples, the average diameter, spheroidization degree, strength, cell culture suitability, and stability of the polymer micro particles were evaluated by the following methods.

**1. Average Diameter**

The average diameter of the polymer micro particles of Examples and Comparative Examples in distilled water was measured using a laser particle size analyzer (Horiba, Partica LA-960).

**2. Spheroidization Degree**

Optical (Olympus, BX53) photographs of the polymer micro particles of Examples and Comparative Examples were taken, and the spheroidization degree was calculated therefrom.

The spheroidization degree according to the present invention was calculated as the average value of the ratio of the longest diameter to the shortest diameter (length-diameter ratio) of 30 arbitrary particles in the optical photograph.

At this time, it means that as the spheroidization degree value is closer to 1, it is closer to a sphere.

3. Strength

For the polymer micro particles of Examples and Comparative Examples, the strength of micro particles was measured using a texture analyzer equipment. 30 micro particles swollen with distilled water for 24 hours were placed as a single layer in the area under the flat cylindrical probe of the equipment equipped with a 5 N load cell. The initial trigger force was set to 1 mN, and the particles were compressed at a rate of 1 mm/s. The force when deformed to a level of 25% of the average particle diameter was defined as the compressive force.

The average compressive strength was calculated by dividing the compressive force by 30, which is the number of micro particles to be measured.

## 4. Cell Culture Suitability

A 6 well plate was filled with cell culture medium, to which polymer micro particles and cells were added, and the cells were cultured by plate-rocking. At this time, the temperature of the culture medium was maintained at 37°C. After culturing for 3 days, the number of cells cultured in polymer micro particles was confirmed.

At this time, the cell culture suitability was evaluated according to the following criteria.

Suitabble: when the number of cultured cells relative to the number of injected cells is 100% or more

Unsuitable: when the number of cultured cells relative to the number of injected cells is less than 100%, or micro particles are decomposed during culture

## 5. Stability

Using a high temperature and high pressure sterilizer (Autoclave), the stability of polymer micro particles contained in phosphate-buffered saline solution during sterilization and particle stability following long-term culture were evaluated based on the following criteria.

Suitable: when the weight reduction rate of the dried polymer micro particles before and after using an autoclave is 20% or less

Unsuitable: when the weight reduction rate of the dried polymer micro particles before and after using an autoclave exceeds 20%

[Table 1]

| Category | Average diameter ($\mu$m) | Spheroidization degree | Average compressive strength (mN) | Cell culture suitability | Stability |
|---|---|---|---|---|---|
| Example 1 | 317±2 1 | 0.95±0.0 8 | 2.10 | Suitable | Suitable |
| Example 2 | 346±2 6 | 0.96±0.1 3 | 1.37 | Suitable | Suitable |
| Example 3 | 319±3 2 | 0.95±0.0 5 | 0.37 | Suitable | Suitable |
| Comparative Example 1 | 385±1 3 | 0.98±0.0 9 | 0.23 | Suitable | Unsuitable |
| Comparative Example 2 | 193±21 | 0.96±0.03 | 0.10 | Unsuitable | Unsuitable |
| Comparative Example 3 | Particle production is impossible | | | | |
| Comparative Example 4 | Particle production is impossible | | | | |

(continued)

| Category | Average diameter ($\mu$m) | Spheroidization degree | Average compressive strength (mN) | Cell culture suitability | Stability |
|---|---|---|---|---|---|
| Comparative Example 5 | Particle production is impossible | | | | |
| Comparative Example 6 | 301$\pm$48 | 0.94$\pm$0.11 | 0.29 | Unsuitable | Suitable |
| Comparative Example 7 | 194$\pm$17 | 0.95$\pm$0.04 | 0.12 | Unsuitable | Unsuitable |

As shown in Table 1, it could be confirmed that in the polymer micro particles of Examples, not only the number of cultured cells relative to the number of cells injected first is 100% or more, which is suitable for cell culture, but also the weight reduction rate of the dried polymer micro particles before and after autoclave treatment is 20% or less, which is suitable for sterilization and long-term culture. In addition, it could be confirmed that in the polymer micro particles of Examples, the average compressive strength appears to be 0.37 mN or more, whereby excellent mechanical properties can be realized and, at the same time, the ratio of particles exhibiting a high crosslinking density is high. That is, it was confirmed that the polymer micro particles of Examples are suitable for cell culture, sterilization treatment, and long-term culture, and also realizes excellent crosslinking density and mechanical properties. On the other hand, it could be confirmed that in the polymer micro particles of Comparative Example 1, not only the weight reduction rate of the dried polymer micro particles before and after the autoclave treatment appears to be more than 20%, which is unsuitable for sterilization and long-term culture, but also the average compressive strength is 0.23 mN, whereby poor mechanical properties are exhibited, and the ratio of particles exhibiting low crosslinking density is high.

In addition, it was observed that in the polymer micro particles of Comparative Example 2, the polymer that could not be crosslinked in the distilled water washing process is dissolved and the particles are contracted. Further, it could be confirmed that not only the micro particles are decomposed during cell culture, and the number of cultured cells relative to the number of injected cells is less than 100%, which is unsuitable for cell culture, but also the weight reduction rate of the dried polymer micro particles before and after autoclave treatment appears to be more than 20%, which is unsuitable for the sterilization treatment and long-term culture. Further, it could be confirmed that the average compressive strength was 0.1 mN, indicating inferior mechanical properties.

Further, it could be confirmed that in the polymer micro particles of Comparative Examples 3 to 5, the concentration of the aqueous solution in which the biocompatible polymer was dissolved is adjusted to the same level as in Example 1, unlike Comparative Example 1, whereby polymer micro particles are not formed, and in the case of Examples, polymer micro particles can be formed even at a low biocompatible polymer concentration.

It could be confirmed that the polymer micro particles of Comparative Example 6 exhibited an average compressive strength of 0.29 mN in distilled water due to chemical crosslinking by the crosslinking agent, but by using alginate and cellulose having no cell adhesion as biocompatible polymers, it is unsuitable for cell culture. Further, as a crosslinking reaction proceeds using calcium ions ($Ca^{2+}$) as metal ions, it can react reversibly with calcium ions present in the cell culture medium, so that the particle strength can be lowered during cell culture.

It could be confirmed that the polymer micro particles of Comparative Example 7 are produced only through ion crosslinking, some micro particles are degraded during sterilization and cell culture, making them unsuitable for cell culture.

**<Experimental Example 2>**

The physical properties of the micro carriers for cell culture produced in Example 1 and Comparative Example 8 were measured by the following method and are described in Table 2 below.

**6. Thickness of Coating layer**

The thickness of the coating layer formed on the surface of the polymer micro particles in the micro carrier for cell culture was measured through a cross-sectional TEM image.

**7. Cell Adhesion**

A culture medium containing mesenchymal stem cells (density: 1.05 g/cm$^3$) was filled in a 100 mL vertical wheel bioreactor (PBS), and the micro carrier for cell culture was injected into the culture medium and stirred. After culturing at 37°C for 7 days, the number of cells cultured in a cell culture micro carrier was confirmed. Then, the cell adhesion of the micro carrier was evaluated by comparing it with the number of initially injected cells as shown in the following Equation.

$$\text{Cell adhesion} = (\text{Number of cells after charging the micro carrier into the cell}$$
$$\text{culture medium and culturing at } 37°C \text{ for 7 days } / \text{ Number of cells initially contained}$$
$$\text{in the cell culture medium}) \times 100.$$

[Table 2]

| Category | Coating layer thickness ($\mu$m) | Cell adhesion (%) |
|---|---|---|
| Example 1 | 0.1 | 2900 |
| Comparative Example 8 | No coating layer | 1900 |

As shown in Table 2, it could be confirmed that as the micro carrier for cell culture of Example 1 includes a cell adhesion material coating layer of 0.1 $\mu$m on the surface of the polymer micro particles, the cell adhesion is remarkably improved as compared to Comparative Example 8.

**Claims**

1. A micro carrier comprising:

   polymer micro particles having a core-shell structure which comprises: a core containing a first biocompatible polymer, a metal ion, and an organic crosslinking agent containing at least one reactive functional group; and a shell surrounding the whole or a part of the core and containing a second biocompatible polymer, a metal ion, and an organic crosslinking agent containing at least one reactive functional group, and a cell adhesion-inducing layer formed on the surface of the polymer micro particles.

2. The micro carrier according to claim 1 wherein:

   the core comprises a polymer matrix in which the first biocompatible polymer is crosslinked via a metal ion and an organic crosslinking agent containing at least one reactive functional group, and the shell comprises a polymer matrix in which the second biocompatible polymer is crosslinked via a metal ion and an organic crosslinking agent containing at least one reactive functional group.

3. The micro carrier according to claim 1 wherein:

   the first biocompatible polymer comprises hyaluronic acid, and the second biocompatible polymer comprises gelatin.

4. The micro carrier according to claim 2 wherein:
   the core comprises a polymer matrix, in which hyaluronic acid is crosslinked via a metal ion and an organic crosslinking agent containing at least one reactive functional group, in an amount of more than 50% by volume with respect to the total volume of the polymer matrix contained in the core.

5. The micro carrier according to claim 2 wherein:
   the shell comprises a polymer matrix, in which gelatin is crosslinked via a metal ion and an organic crosslinking agent containing at least one reactive functional group, in an amount of more than 50% by volume with respect to the total volume of the polymer matrix contained in the shell.

6. The micro carrier according to claim 1 wherein:

the polymer micro particles have an average diameter of 1 $\mu$m or more in distilled water.

7. The micro carrier according to claim 1 wherein:
   a thickness of the shell is 95% or less of the longest diameter of the polymer micro particles, based on the cross section having the longest diameter of the polymer micro particles.

8. The micro carrier according to claim 1 wherein:
   the organic crosslinking agent containing at least one reactive functional group comprises a crosslinking agent having 1 to 30 carbon atoms containing at least one reactive functional group.

9. The micro carrier according to claim 1 wherein:
   the polymer micro particles have an average compressive strength of 0.1 mN or more when deformed to a level of 25% of the average particle diameter.

10. The micro carrier according to claim 1 wherein:
    the polymer micro particles has a spheroidization degree, which is the ratio of the longest diameter to the shortest diameter of arbitrary particles in an optical photograph, of 0.9 or more and 1.0 or less.

11. The micro carrier according to claim 1 wherein:
    the cell adhesion-inducing layer comprises one or more cell adhesion materials selected from the group consisting of gelatin, collagen, fibronectin chitosan, polydopamine, poly L-lysine, vitronectin, peptide containing RGD, acrylic polymer containing RGD, lignin, cationic dextran and derivatives thereof.

12. The micro carrier according to claim 1, wherein:
    the cell adhesion-inducing layer has a layer thickness of 1 nm to 10,000 nm.

13. The micro carrier according to claim 1, wherein:
    the micro carriers have an average diameter of 1 $\mu$m to 1000 $\mu$m.

14. The micro carrier according to claim 1, wherein:
    the micro carrier has a cell adhesion of 2000% or more as calculated according to the following Equation:

[Equation]

$$\text{Cell adhesion} = (\text{Number of cells after charging the micro carrier into the cell culture medium and culturing at } 37°C \text{ for 7 days } / \text{ Number of cells initially contained in the cell culture medium}) \times 100.$$

15. The micro carrier according to claim 1, wherein:
    the micro carrier is a micro carrier for cell culture.

16. A cell composite comprising:

    the micro carrier of claim 1; and
    cells adhered onto the surface of the micro carrier.

17. A medical composition comprising any one of the micro carrier of claim 1 or the cell composite of claim 16.

18. A cosmetic composition comprising any one of the micro carrier of claim 1 or the cell composite of claim 16.

19. A medical article comprising the medical composition of claim 17 .

20. A cosmetic article comprising the cosmetic composition of claim 18

【FIG. 1】

【FIG. 2】

【FIG. 3】

### INTERNATIONAL SEARCH REPORT

| International application No. |
| --- |
| **PCT/KR2022/012636** |

**A. CLASSIFICATION OF SUBJECT MATTER**

**C12M 1/12**(2006.01)i; **C12N 11/06**(2006.01)i; **C12N 11/08**(2006.01)i; **A61K 9/16**(2006.01)i; **A61K 35/12**(2006.01)i; **A61K 8/02**(2006.01)i; **A61K 8/73**(2006.01)i; **A61K 8/65**(2006.01)i; **A61K 8/98**(2006.01)i

According to International Patent Classification (IPC) or to both national classification and IPC

**B. FIELDS SEARCHED**

Minimum documentation searched (classification system followed by classification symbols)

C12M 1/12(2006.01); A61K 47/36(2006.01); A61K 8/64(2006.01); A61K 8/97(2006.01); A61K 9/16(2006.01); A61K 9/50(2006.01); C12N 11/08(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 히알루론산(hyaluronic acid), 콜라겐(collagen), 코어-쉘(core-shell), 마이크로 캐리어(micro carrier), 가교제(crosslinking agent)

**C. DOCUMENTS CONSIDERED TO BE RELEVANT**

| Category* | Citation of document, with indication, where appropriate, of the relevant passages | Relevant to claim No. |
| --- | --- | --- |
| Y | KR 10-1999-0037094 A (SS PHARM. CO., LTD.) 25 May 1999 (1999-05-25) See claims 1 and 3; paragraphs [0018] and [0021]-[0025]; and example 41. | 1-20 |
| Y | KR 10-2004-0021615 A (HISAMITSU PHARMACEUTICAL CO., INC.) 10 March 2004 (2004-03-10) See claims 2 and 5; and example 1. | 1-20 |
| Y | KR 10-2021-0011340 A (LG CHEM, LTD.) 01 February 2021 (2021-02-01) See claims 1 and 6; and paragraphs [0083]-[0084]. | 1-20 |
| A | KR 10-2011-0118859 A (KOREA INSTITUTE OF SCIENCE AND TECHNOLOGY) 02 November 2011 (2011-11-02) See entire document. | 1-20 |
| A | KR 10-2201482 B1 (FIUMBIO CO., LTD.) 13 January 2021 (2021-01-13) See entire document. | 1-20 |

☐ Further documents are listed in the continuation of Box C.  ☑ See patent family annex.

| | |
| --- | --- |
| * Special categories of cited documents: | "T" later document published after the international filing date or priority date and not in conflict with the application but cited to understand the principle or theory underlying the invention |
| "A" document defining the general state of the art which is not considered to be of particular relevance | |
| "D" document cited by the applicant in the international application | "X" document of particular relevance; the claimed invention cannot be considered novel or cannot be considered to involve an inventive step when the document is taken alone |
| "E" earlier application or patent but published on or after the international filing date | |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
| --- | --- |
| **01 December 2022** | **02 December 2022** |

| Name and mailing address of the ISA/KR | Authorized officer |
| --- | --- |
| **Korean Intellectual Property Office** **Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2022)

INTERNATIONAL SEARCH REPORT
Information on patent family members

International application No.

**PCT/KR2022/012636**

| Patent document cited in search report | | | Publication date (day/month/year) | Patent family member(s) | | | Publication date (day/month/year) |
|---|---|---|---|---|---|---|---|
| KR | 10-1999-0037094 | A | 25 May 1999 | None | | | |
| KR | 10-2004-0021615 | A | 10 March 2004 | None | | | |
| KR | 10-2021-0011340 | A | 01 February 2021 | EP | 3889252 | A1 | 06 October 2021 |
| | | | | JP | 2022-521518 | A | 08 April 2022 |
| | | | | KR | 10-2467863 | B1 | 16 November 2022 |
| | | | | US | 2022-0081684 | A1 | 17 March 2022 |
| | | | | WO | 2021-015547 | A1 | 28 January 2021 |
| KR | 10-2011-0118859 | A | 02 November 2011 | KR | 10-1270161 | B1 | 31 May 2013 |
| | | | | US | 2011-0263018 | A1 | 27 October 2011 |
| KR | 10-2201482 | B1 | 13 January 2021 | None | | | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- KR 1020210130366